# EUROPÄISCHE PATENTSCHRIFT

(11) **EP 3 636 228 B1**
(45) Veröffentlichungstag und Bekanntmachung des Hinweises auf die Patenterteilung: **03.04.2024**
(21) Anmeldenummer: 19211513.7
(22) Anmeldetag: 24.02.2017
(51) Int. Cl.: A61F 5/01, A61F 2/50, F16F 9/19, F16F 9/20, F16F 9/30, F16F 9/32, F16F 9/512, F16F 9/516

(54) **VORRICHTUNG ZUR STABILISIERUNG VON KÖRPERGELENKEN**
DEVICE FOR STABILISING JOINTS
DISPOSITIF DE STABILISATION DES ARTICULATIONS CORPORELLES

(30) Priorität: 25.04.2016 DE 102016107664
(43) Veröffentlichungstag der Anmeldung: 15.04.2020
(62) Teilanmeldung aus: 17157941.0
(73) Patentinhaber: Betterguards Technology GmbH, 10625 Berlin (DE)
(72) Erfinder: Bichler, Vinzenz, 10777 Berlin (DE); Stumper, Timo, 10963 Berlin (DE); Buschinger, Oscar, 10707 Berlin (DE)
(74) Vertreter: Okoampah, Rene

(56) Entgegenhaltungen:
- EP-A1- 2 842 527
- WO-A1-2013/174989
- WO-A1-2015/177357
- US-A1- 2014 015 176

## Beschreibung

### Technisches Gebiet

Die vorliegende Erfindung betrifft eine Vorrichtung zur Stabilisierung von Körpergelenken, welche eine Aufnahme, wobei die Aufnahme mit einem Füllmedium gefüllt ist, einen ersten Körper zum Interagieren mit dem Füllmedium, wobei der erste Körper in der Aufnahme verschiebbar angeordnet ist, und ein Mittel zum Übertragen einer äußeren Kraft auf den ersten Körper, umfasst.

### Stand der Technik

Es ist bekannt Körpergelenke, Muskeln und Sehnen mittels Vorrichtungen, welche eine adaptive Bewegungsbegrenzung ermöglichen zu stabilisieren. Unter anderem wird das adaptive Verhalten solcher Vorrichtungen dadurch erreicht, dass sich zwei Körper relativ zueinander bewegen, wobei sich zwischen den Körpern ein scherverdickendes Fluid befindet. Die sich gegenüberliegenden Flächen der Körper bilden dabei Scherflächen, welche auf Grund der Relativbewegung Scherkräfte in das scherverdickende Fluid einleiten. Umso größer die Scherkräfte sind umso zähflüssiger verhält sich das scherverdickende Fluid. Ab einer definierten Schergeschwindigkeit erfährt das scherverdickende Fluid einen Schersprung, durch welchen der Grad der Viskosität rapide zunimmt.

Die Vorrichtungen werden zwischen zwei Körperstellen eines Anwenders fixiert. Dabei bildet ein Scherkörper der Vorrichtung eine Aufnahme, welche mit dem scherverdickenden Fluid gefüllt ist. Der andere Scherkörper bildet einen Auszugskörper, welcher in der Aufnahme bewegbar angeordnet ist. Werden über die beiden Körperstellen des Anwenders physiologische Kräfte, das heißt für das entsprechend zu stabilisierende Körperteil unkritische Kräfte, in die Vorrichtung eingeleitet, so wird auf Grund des flüssigen Zustands des scherverdickenden Fluids eine Relativbewegung der Aufnahme und des Auszugskörpers und damit eine Bewegung des zu stabilisierenden Körperteils zugelassen.

Werden hingegen unphysiologische Kräfte, das heißt für das entsprechend zu stabilisierende Körperteil kritische Kräfte, in die Vorrichtung eingeleitet, rufen die von den Scherflächen der Aufnahme und des Auszugskörpers ausgehenden Scherkräfte eine Scherverfestigung des scherverdickenden Fliuds hervor, durch welche eine Relativbewegung zwischen dem Auszugskörper und der Aufnahme nur noch unter sehr hohem Kraftaufwand möglich ist.

Eine solche Vorrichtung ist beispielsweise aus der WO 2013/174989 A1 bekannt, welche eine orthopädische Vorrichtung zur Begrenzung der Bewegung eines zwischen einem ersten und einem zweiten Körperbereich angeordneten Gelenks zeigt.

Bei den bekannten Vorrichtungen erfolgt die Aufrechterhaltung der Scherverfestigung jedoch nur über einen relativ kurzen Zeitraum. Kurz nach dem, der die Scherverfestigung auslösende Impuls, nachlässt, das heißt, sobald die Geschwindigkeit der Krafteinwirkung abnimmt, fällt auch die durch die Vorrichtung bereitgestellte stabilisierende Rückhaltekraft wieder weg.

Dies ist gerade dann problematisch, wenn nach einer abrupten, unphysiologischen Krafteinwirkung auf das zu schützenden Körperteil eine Kraft verbleibt, die deutlich langsamer auf den Körperteil wirkt. Nach dem Wegfallen der schützenden Wirkung der Vorrichtung ist der Körperteil dieser Kraft ungehindert ausgesetzt.

Dies kann beispielsweise im Bereich des Sprunggelenks auftreten. Hier können die mit einer hohen Geschwindigkeit auftretenden Kräfte beim Umknicken zunächst von der Vorrichtung kompensiert werden. Lässt die Wirkung der Vorrichtung jedoch nach, können kleinere, langsamere Bewegungen welche beispielsweise durch das Körpergewicht hervorgerufen werden nicht unterbunden werden, da das scherverdickende Medium bei diesen geringen Geschwindigkeiten nicht verfestigt wird. Das Fortschreiten der Umknickbewegung kann die Folge sein.

EP 2 842 527 A1 zeigt eine tragbare Haltungsunterstützungsvorrichtung und offenbart sämtliche im Oberbegriff des Anspruchs 1 genannten technischen Merkmale.

### Darstellung der Erfindung

Ausgehend von dem bekannten Stand der Technik ist es eine Aufgabe, eine verbesserte Vorrichtung zur Stabilisierung von Körpergelenken anzugeben, insbesondere soll die schützende Wirkung der Vorrichtung möglichst lange anhalten.

Diese Aufgabe wird mittels einer Vorrichtung mit den Merkmalen des Anspruchs 1 gelöst. Vorteilhafte Ausgestaltungen ergeben sich aus den Unteransprüchen.

Entsprechend wird eine Vorrichtung zur Stabilisierung von Körpergelenken angegeben, welche eine Aufnahme, wobei die Aufnahme mit einem Füllmedium gefüllt ist, einen ersten Körper zum Interagieren mit dem Füllmedium, wobei der erste Körper in der Aufnahme verschiebbar angeordnet ist, und ein Kraftübertragungsmittel zum Übertragen einer äußeren Kraft auf den ersten Körper, umfasst. Erfindungsgemäß ist ein zweiter Körper zum Interagieren mit dem Füllmedium in der Aufnahme verschiebbar angeordnet, wobei der zweite Körper über ein Kopplungselement elastisch mit dem ersten Körper gekoppelt ist. Ferner weist der zweite Körper und/oder der erste Körper mindestens eine Durchlassöffnung auf, durch welche das Füllmedium strömen kann. Die Durchlassöffnung in dem zweiten Körper und/oder dem ersten Körper stellt einen zusätzlichen Fließweg für das Füllmedium bereit, durch welchen das Füllmedium fließen kann, solange zwischen dem ersten Körper und dem zweiten Körper ein Abstand besteht.

Darüber hinaus bildet der erste Körper einen Ventilkörper und der zweite Körper einen Ventilsitz, so dass ein Fluss des Mediums durch die Durchlassöffnung in Abhängigkeit von der Ventilstellung zugelassen oder unterbunden werden kann. In der geschlossenen Ventilstellung kann das Füllmedium nur noch im Bereich zwischen der Aufnahme und der Scherflächen des ersten Körpers und des zweiten Körpers strömen, sofern es der Grad der Scherspannungserhöhung zulässt.

Dadurch ist es möglich, auf den ersten Körper einwirkende äußere Kräfte über das Kopplungselement auf den zweiten Körper zu übertragen. Entsprechend ist der erste Körper dazu in der Lage, den zweiten Körper mittels des Kopplungselements durch das Füllmedium zu schieben und/oder zu ziehen.

Dabei sind die Scherflächen des ersten Körpers und des zweiten Körpers derart konfiguriert, dass beim Einwirken einer äußeren Kraft auf den ersten Körper mit einer Geschwindigkeit im physiologischen Bereich, beide Körper durch das Füllmedium bewegt werden können. Zusätzlich ist das Kopplungselement derart konfiguriert, dass es beim Einwirken einer äußeren Kraft auf den ersten Körper, im Bereich einer physiologischen Geschwindigkeit, eine Kraft auf den zweiten Körper überträgt, so dass dieser gemeinsam mit dem ersten Körper durch das Füllmedium bewegt werden kann.

Erreicht die über den ersten Körper und das Kopplungselement auf den zweiten Körper wirkende Kraft eine kritische Geschwindigkeit, das heißt eine unphysiologische Geschwindigkeit, kommt es an den Scherflächen des zweiten Körpers zu einer Erhöhung der Scherspannung. Infolge dieser Scherspannungserhöhung übt das Füllmedium eine Widerstandskraft gegen die Bewegung des zweiten Körpers aus.

Erreicht diese Widerstandskraft einen Betrag gleich oder größer der von dem Kopplungselement auf den zweiten Körper wirkenden Kraft, kann der zweite Körper nicht weiter durch das Füllmedium bewegt werden und wird von diesem blockiert.

Auch im Bereich der Scherflächen des ersten Körpers tritt eine Scherspannungserhöhung auf, sobald die Geschwindigkeit, mit welcher der erste Körper mittels der äußeren Kraft durch das Füllmedium bewegt wird, einen kritischen Wert, das heißt eine unphysiologische Geschwindigkeit, erreicht. Von dem Füllmedium geht dann eine Widerstandskraft aus, welche der auf den ersten Körper wirkenden äußeren Kraft entgegenwirkt. Dabei kann der erste Körper solange durch das Füllmedium bewegt werden, bis die von dem Füllmedium ausgehende Widerstandskraft, der auf den ersten Körper wirkenden äußeren Kraft gleicht.

Für den Fall, dass der zweite Körper durch die von dem Füllmedium ausgehende Widerstandskraft blockiert wird, während die auf den ersten Körper wirkende äußere Kraft größer als die von dem Füllmedium ausgehende Widerstandskraft ist, kann der erste Körper relativ zu dem zweiten Körper bewegt werden.

Wirkt dabei die äußere Kraft derart auf den ersten Körper, dass sich der Abstand zwischen dem ersten Körper und dem zweiten Körper verringert, kann der Abstand zwischen dem ersten Körper und dem zweiten Körper geschlossen werden. In diesem Zustand wirken der äußeren, an dem ersten Körper anliegenden Kraft sowohl die an den Scherflächen des ersten Körpers erzeugte Widerstandskraft als auch die an den Scherflächen des zweiten Körpers erzeugte Widerstandskraft entgegen. Durch die in Summe größere Scherfläche kann die Scherspannungserhöhung schneller und intensiver eintreten, so dass die Vorrichtung insgesamt schneller und mit höheren Rückhaltekräften reagiert. Dies ist vor allem vor dem Hintergrund vorteilhaft, eine Schutzwirkung nach einer Zeitdauer bereitzustellen, die kürzer ist als die Reaktionszeit der Muskeln des Anwenders.

Bewegungen von Körpergelenken lassen sich dadurch schnell abbremsen. Des Weiteren lässt sich dadurch die Scherspannungserhöhung des Füllmedium im Bereich der in Summe vergrößerten Scherfläche länger aufrechterhalten. Um den ersten Körper und den zweiten Körper in dem aneinanderliegenden Zustand zu bewegen, ist eine deutlich größere äußere Kraft notwendig, als bei der Bewegung des ersten Körpers und den zweiten Körpers in einem voneinander beabstandeten Zustand. Somit kann die Scherspannungserhöhung im aneinander liegenden Zustand auch bei deutlich langsamer wirkenden Kräften aufrechterhalten werden.

Dies ist beispielsweise für Anwendungen der Vorrichtungen im Bereich des Sprunggelenks vorteilhaft, da nach einem Umknicken meist das gesamte Körpergewicht auf dem Knöchel lastet. Entsprechend lässt sich mittels der Vorrichtung sowohl der durch die Umknickbewegung auf das Gelenk wirkenden Kraft als auch der anschließenden, durch das Körpergewicht auf das Gelenk langsamer wirkenden Kraft, entgegenwirken. Entsprechend kann die Vorrichtung auch dann noch eine Haltekraft bereitstellen, wenn der primäre Impuls, das heißt die einwirkende Kraft, mit hoher Geschwindigkeit nachlässt.

In einer bevorzugten Ausgestaltung weisen die Scherfläche des ersten Körpers und die Scherfläche des zweiten Körpers eine unterschiedliche Größe auf. Dabei sind als Scherflächen des ersten Körpers und des zweiten Körpers die Oberflächen definiert, gegenüber welchen das Füllmedium eine relative Strömungsbewegung ausführt, wenn der erste Körper und/oder der zweite Körper aufgrund einer äußeren Kraft bewegt werden. Beim Erreichen einer kritischen Schergeschwindigkeit, welche auf die auf den ersten Körper wirkende äußere Kraft zurückzuführen ist, kommt es in dem Füllmedium in der Umgebung der Scherflächen zu einer Scherspannungserhöhung.

Durch die Wahl von unterschiedlich großen Scherflächen bezüglich des ersten Körpers gegenüber dem zweiten Körper ist es möglich, die Interaktion des ersten Körpers und des zweiten Körpers mit dem Füllmedium und somit die jeweils auf die Körper wirkende Widerstandskraft, bei gegebener anliegender äußeren Kraft, festzulegen. Dadurch kann ein unterschiedliches Verhalten des ersten Körpers und des zweiten Körpers bei einer auf den ersten Körper wirkenden äußeren Kraft erreicht werden.

In einer bevorzugten Ausführungsform ist die Scherfläche des ersten Körpers kleiner als die Scherfläche des zweiten Körpers. Dadurch ist es möglich, dass, wenn der erste Körper und der zweite Körper aufgrund einer auf den ersten Körper aufgebrachten äußeren Kraft durch das Füllmedium bewegt werden, auf den zweiten Körper eine größere Widerstandskraft wirkt, als auf den ersten Körper. Entsprechend weist der zweite Körper eine kritische Schergeschwindigkeit auf, die kleiner ist als die kritische Schergeschwindigkeit des ersten Körpers. Erreichen die Körper durch das Anliegen der äußeren Kraft die kritische Schergeschwindigkeit des zweiten Körpers, wird der zweite Körper aufgrund der Scherspannungserhöhung des Füllmediums im Bereich der Scherflächen des zweiten Körpers blockiert, während sich der erste Körper weiterhin durch das scherverdickende Medium bewegen kann. Anders ausgedrückt ist die auf den zweiten Körper wirkende Widerstandskraft beim Erreichen der kritischen Schergeschwindigkeit des zweiten Körpers gleich der von dem elastischen Kopplungselement ausgehenden Kraft, während die auf den ersten Körper wirkende Widerstandskraft noch kleiner als die auf den ersten Körper wirkende äußere Kraft ist.

In der Folge wird der erste Körper auf den zweiten Körper zubewegt, bis der Abstand zwischen dem ersten Körper und dem zweiten Körper geschlossen ist. Im geschlossenen Zustand steht der äußeren Kraft die Widerstandskraft entgegen, welche sich aus der Interaktion der Summe der Scherflächen des ersten Körpers und des zweiten Körpers mit dem Füllmedium ergibt. Die Scherspannungserhöhung tritt nun bereits bei geringeren Geschwindigkeiten ein, beziehungsweise bleibt die Scherspannungserhöhung bei weiter wirkenden Kräften mit geringen Geschwindigkeiten aufrechterhalten, so dass die Vorrichtung insgesamt schneller und mit höherer Rückhaltekraft reagiert.

In einer Weiterbildung ist ein Spaltmaß zwischen dem ersten Körper und der Aufnahme unterschiedlich zu einem Spaltmaß zwischen dem zweiten Körper und der Aufnahme. Das Spaltmaß stellt die kleinste Querschnittfläche dar, durch welche das Füllmediums relativ gegenüber dem ersten Körper bzw. dem zweiten Körper strömen kann. Die Querschnittfläche liegt dabei in einer Ebene senkrecht zu einer Hauptbewegungsrichtung des ersten Körpers und des zweiten Körpers.

Über das Spaltmaß lässt sich die kritische Schwergeschwindigkeit des ersten Körpers und des zweiten Körpers beeinflussen. Indem der erste Körper und der zweite Körper unterschiedlich weit von der seitlichen Wandung der Aufnahme entfernt sind, ist es möglich, dass an dem ersten Körper und dem zweiten Körper das Füllmedium mit unterschiedlichen Geschwindigkeiten vorbeiströmen kann.

Dadurch kann erreicht werden, dass sich die kritische Schergeschwindigkeit des ersten Körpers von der kritischen Schergeschwindigkeit des zweiten Körpers unterscheidet.

In einer weiter bevorzugten Ausführungsform ist das Spaltmaß zwischen dem ersten Körper und der Aufnahme größer als das Spaltmaß zwischen dem zweiten Körper und der Aufnahme. Dies kann beispielsweise durch eine sich konisch verjüngende Aufnahme erreicht werden. Dadurch ist es möglich, dass, wenn der erste Körper und der zweite Körper aufgrund einer auf den ersten Körper aufgebrachten äußeren Kraft durch das Füllmedium bewegt werden, auf den zweiten Körper eine größere Widerstandskraft wirkt, als auf den ersten Körper. Entsprechend weist der zweite Körper eine kritische Schergeschwindigkeit auf, die kleiner ist als die kritische Schergeschwindigkeit des ersten Körpers. Erreichen die Körper durch das Anliegen der äußeren Kraft die kritische Schergeschwindigkeit des zweiten Körpers, wird der zweite Körper aufgrund der Scherspannungserhöhung des Füllmediums im Bereich der Scherflächen des zweiten Körpers blockiert, während sich der erste Körper weiterhin durch das Füllmedium bewegen kann. Anders ausgedrückt ist die auf den zweiten Körper wirkende Widerstandskraft beim Erreichen der kritischen Schergeschwindigkeit des zweiten Körpers gleich der von dem elastischen Kopplungselement ausgehenden Kraft, während die auf den ersten Körper wirkende Widerstandskraft noch kleiner als die auf den ersten Körper wirkende äußere Kraft ist.

In einer weiter bevorzugten Weiterbildung umfasst das Kopplungselement mindestens ein Federelement. Dabei kann das Federelement eine Druckfeder, eine Zugfeder und/oder ein elastisches Polymer umfassen. Anhand der Federkraft des Federelements kann das Einsetzen des Dilatanzsprungs der Vorrichtung definiert werden. Entsprechend wird der zweite Körper in seiner Bewegung blockiert, wenn die auf die Scherspannungserhöhung des Füllmedium zurückzuführende Widerstandskraft, welche auf den zweiten Körper wirkt, gleich der Federkraft des Federelements ist.

Nachdem der zweite Körper aufgrund der Scherspannungserhöhung des Füllmediums blockiert ist, wird das Federelement durch die fortschreitende Bewegung des ersten Körpers hin zu dem zweiten Körper zusammengedrückt.

In einer weiter bevorzugten Ausgestaltung ist das Kopplungselement aus einem Material mit einem temperaturabhängigen Elastizitätsmodul gefertigt. Dadurch können Temperaturschwankungen, welche sich auf das Verhalten des Füllmediums auswirken, kompensiert werden. Schwankungen der Temperatur der Vorrichtung können beispielsweise durch die Körpertemperatur des Anwenders oder die Umgebungstemperatur hervorgerufen werden. Da die Viskosität des Füllmediums bei ansteigender Temperatur abnimmt, kann es zu Unregelmäßigkeiten beim Einsetzen des Dilatanzsprungs kommen. Das heißt, bei höheren Temperaturen setzt der Dilatanzsprung später ein als bei niedrigen Temperaturen.

Durch den Einsatz eines Kopplungselements mit einem temperaturabhängigen Elastizitätsmodul kann den temperaturbedingten Schwankungen des Einsetzens des Dilatanzsprungs entgegengewirkt werden. Dabei kann die Temperaturabhängigkeit des Kopplungselements derart definiert werden, dass der Elastizitätsmodul des Kopplungselements mit steigender Temperatur abnimmt. Dies hat zur Folge, dass, wenn sich mit ansteigender Temperatur die erforderliche kritische Schergeschwindigkeit erhöht, mit welcher sich der zweite Körper durch das Füllmedium bewegt, um von diesem abgebremst zu werden, zugleich die von dem elastischen Kopplungselement ausgehende Kraft reduziert. Entsprechend sinkt mit steigender Temperatur die auf den zweiten Körper wirkende Widerstandskraft, die nötig ist, um den zweiten Körper zu blockieren. Beispielsweise kann das Kopplungselement in Form eines Federelements ausgelegt werden, wobei die Federkonstante in Abhängigkeit der Temperatur variiert, wodurch ein Angleichen der unterschiedlich vorliegenden Viskosität des Füllmediums erfolgen kann.

Insgesamt lassen sich somit auf die Vorrichtung wirkende Temperaturschwankungen kompensieren, so dass sich der erste Körper auch bei unterschiedlich vorherrschenden Temperaturen nahezu homogen verhält, wenn die äußere Kraft indirekt auf den zweiten Körper wirkt.

In einer bevorzugten Ausgestaltung ist die Durchlassöffnung mittels des ersten Körpers und/oder des zweiten Körpers verschließbar, so dass ein Fluss des Mediums durch die Durchlassöffnung unterbunden werden kann. Wird der erste Körper durch die äußere Kraft soweit auf den zweiten Körper zu bewegt, dass der Abstand zwischen den Körpern geschlossen ist, ist auch die Durchlassöffnung geschlossen, so dass das Füllmedium nur noch zwischen den Außenflächen des ersten Körpers und des zweiten Körpers und der Innenfläche der Aufnahme fließen kann, sofern es der Grad der Scherverfestigung zulässt. Gleiches gilt auch in umgekehrter Weise für den Fall, dass der erste Körper mindestens eine Durchlassöffnung aufweist.

In einer Weiterbildung ist das Kraftübertragungsmittel zum Übertragen der äußeren Kraft einstückig mit dem ersten Körper ausgebildet. In diesem Fall bildet der erste Körper gemeinsam mit dem Kraftübertragungsmittel einen Auszugskörper, wobei das außerhalb der Vorrichtung liegende Ende des Kraftübertragungsmittels mit einem Körperteil des Anwenders verbunden ist und die Aufnahme mit einem anderen Körperteil des Anwenders verbunden ist.

In einer weiter bevorzugten Ausführungsform kann der erste Körper mittels des Kopplungselements eine Druckkraft und/oder eine Zugkraft auf den zweiten Körper ausüben. Wird der erste Körper durch das Einwirken einer äußeren Kraft von dem zweiten Körper weg bewegt, kann der zweite Körper über das elastische Kopplungselement von dem ersten Körper mitgezogen werden. Wird der erste Körper hingegen durch die äußere Kraft in Richtung des zweiten Körpers bewegt, kann der erste Körper über das elastische Kopplungselement den zweiten Körper in die gleiche Bewegungsrichtung drücken.

In einer weiter bevorzugten Ausführungsform sind die Größe der Scherfläche des ersten Körpers und die Größe der Scherfläche des zweiten Körpers derart konfiguriert, dass wenn die äußere Kraft mit einer Geschwindigkeit unterhalb eines Schwellwerts auf den ersten Körper wirkt, der erste Körper und der zweite Körper nahezu gleichmäßig durch das Füllmedium bewegbar sind, und dass wenn die äußere Kraft mit einer Geschwindigkeit gleich oder größer des Schwellwerts auf den ersten Körper wirkt, der erste Körper und der zweite Körper relativ zueinander bewegbar sind. Dabei stellt der Schwellwert der Geschwindigkeit den Wert dar, bei welchem die Widerstandskraft durch die Scherspannung genauso groß ist, wie die Widerstandskraft des elastischen Kopplungselements.

In einer Weiterbildung ist das Füllmedium ein Fluid. Beispielsweise können Newtonsche Flüssigkeiten wie zum Beispiel Silikonöl als Füllmedium verwendet werden. Newtonsche Flüssigkeiten zeigen ein lineares Verhalten, das heißt die Scherspannung steigt über die Schergeschwindigkeit linear an. Dadurch ist eine Geschwindigkeitsabhängige Dämpfung möglich.

In einer weiter bevorzugten Weiterbildung ist das Füllmedium scherverdickend. Beispielsweise kann das Füllmedium ein scherverdickendes Fluid sein. Unter scherverdickenden Fluiden sind im Allgemeinen und insbesondere in der vorliegenden Anmeldung Copolymerdispersionen zu verstehen, wie sie beispielsweise in der DE 30 25 562 A1, der DE 34 33 085 A1 und der DE 39 17 456 A1 gezeigt sind. Die Dispersionen setzen sich beispielsweise aus Emulsionscopolymerisaten und Metallsalzen zusammen. Die Emulsionscopolymerisate können beispielsweise aus 1 - 10 Gew.-% monoolefinisch ungesättigten Mono- und/oder Dicarponsäuren, wie Acryl-, Methacryl-, Malein- und/oder Fumarsäure, 99 - 90 Gew.-% andere olefinisch ungesättigte Monomere, wie Styrol, C1-C6-Alkylacrylate, wie Methylmethacrylat, und 5 - 30 Gew.-% eines Carponsäurealylester-Monomers mit zwei oder mehr copolymerisierbaren Doppelbindungen, wie beispielsweise Diallylphthallat polymerisiert werden.

Als Metallsalze werden in der Regel 0,1 bis 30 Gew.-% bezogen auf die Copolymerisate an Metalloxiden, -Hydroxiden, -Halogeniden, - Carbonaten, -Hydrogensulfaten, -Sulfaten und/oder-Phosphaten zugesetzt. Weiterhin enthalten die scherverdickende Flüssigkeiten Verdünnungsmittel wie Alkohole, Glykole, Di- und Triglykole, Formamide und/oder Wasser. Für eine detailliertere Zusammensetzung des scherverdickenden Fluids wird auf DE 30 25 562 A1, DE 39 17 456 A1 sowie die auf EP 1 443 097 A1 verwiesen. Darüber hinaus können Scherverdickende Fluide auch einfache Dispersionen sein, welche ab einem bestimmten Feststoffanteil scherverdickende Eigenschaften aufweisen.

### Kurze Beschreibung der Figuren

Bevorzugte weitere Ausführungsformen und Aspekte der vorliegenden Erfindung werden durch die nachfolgende Beschreibung der Figuren näher erläutert. Dabei zeigen:
- Fig. 1A: eine perspektivische Ansicht einer Vorrichtung zur Stabilisierung von Körpergelenken,
- Fig. 1B: eine perspektivische Schnittansicht der Vorrichtung aus Fig. 1A in einem Ausgangszustand,
- Fig. 1C: eine Schnittansicht der Vorrichtung aus Fig. 1A in einem Ausgangszustand,
- Fig. 2A: eine perspektivische Schnittansicht der Vorrichtung aus Fig. 1A in einem Haltezustand,
- Fig. 2B: eine Schnittansicht der Vorrichtung aus Fig. 1A in einem Haltezustand,
- Fig. 3A: eine Schnittansicht der Vorrichtung aus Fig. 1A entlang der Schnittlinie A-A aus Figur 2B,
- Fig. 3B: eine Schnittansicht der Vorrichtung aus Fig. 1A entlang der Schnittlinie C-C aus Figur 2B,
- Fig. 4A: eine perspektivische Ansicht des ersten Körpers und des zweiten Körpers in einem Ausgangszustand,
- Fig. 4B: eine perspektivische Ansicht des ersten Körpers und des zweiten Körpers in einem komprimierten Zustand,
- Figur 5: eine schematische Schnittansicht einer Vorrichtung zur Stabilisierung von Körpergelenken wobei die Körper unterschiedlich große Spaltmaße aufweisen,
- Figur 6: eine schematische Schnittansicht einer Vorrichtung zur Stabilisierung von Körpergelenken wobei die Körper unterschiedlich große Scherflächen aufweisen,
- Figur 7: eine schematische Schnittansicht einer Vorrichtung zur Stabilisierung von Körpergelenken wobei die Aufnahme eine Stufe aufweist, und
- Figur 8: eine schematische Schnittansicht einer Vorrichtung zur Stabilisierung von Körpergelenken wobei die Aufnahme eine konische Form aufweist.

### Detaillierte Beschreibung bevorzugter Ausführungsbeispiele

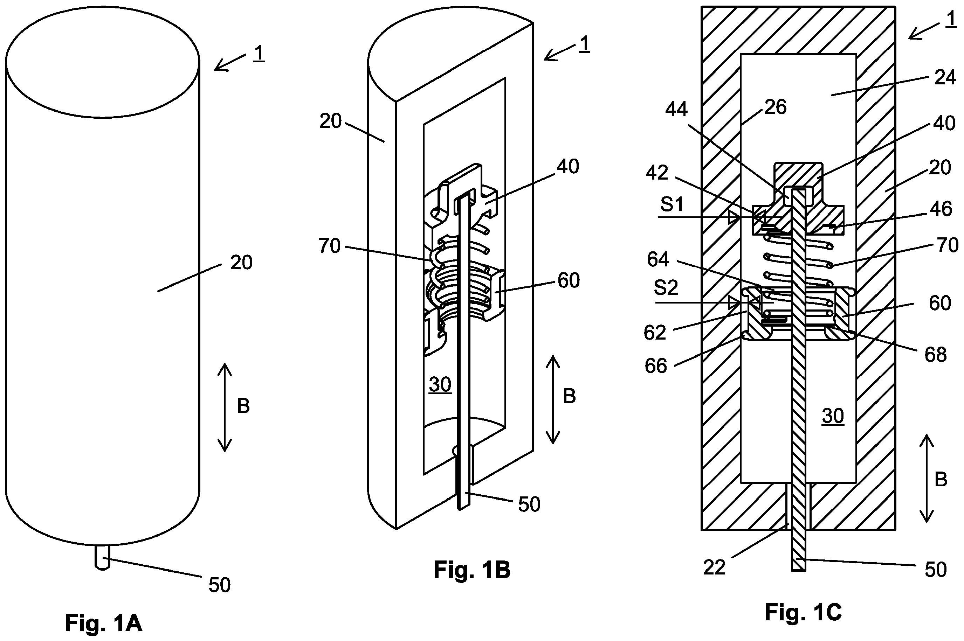

Figur 1A ist eine perspektivische Ansicht einer Vorrichtung 1 zur Stabilisierung von Körpergelenken zu entnehmen. Aus einer zylindrischen Aufnahme 20 ragt ein Kraftübertragungsmittel 50 heraus. Dabei kann die Aufnahme an einem Körperteil eines Anwenders befestigt werden und das Kraftübertragungsmittel 50 an einem anderen Körperteil des Anwenders. Die Richtung B stellt die Bewegungsrichtung der Vorrichtung dar. Alternative kann die Aufnahme auch quaderförmig ausgebildet sein.

Figuren 1B und 1C ist das Innere der Vorrichtung 1, welche sich in einem Ausgangszustand befindet, zu entnehmen. Die Vorrichtung 1 umfasst eine Aufnahme 20, welche an einem Bereich des Körpers eines Anwenders befestigt werden kann. Die Aufnahme 20 weist eine Öffnung 22 auf, durch welche ein Kraftübertragungsmittel 50 in den Innenraum der Vorrichtung 20 hineinragt. Das Ende des Kraftübertragungsmittels 50, welches außerhalb der Vorrichtung 20 liegt, kann an einem anderen Bereich des Körpers des Anwenders befestigt werden.

Bewegt sich der Körperbereich des Anwenders, an welchem die Aufnahme 20 befestigt ist, relativ zu dem Körperbereich des Anwenders, an dem das Kraftübertragungsmittel 50 angeordnet ist, so bewegt sich das Kraftübertragungsmittel 50 relativ zu der Aufnahme 20. Insbesondere kann sich das Kraftübertragungsmittel 50 in einer Hauptbewegungsrichtung B weiter in die Aufnahme 20 hinein oder weiter aus der Aufnahme 20 heraus bewegen. Die Aufnahme der Vorrichtung ist aus Kunststoff gefertigt. Unter anderem könne auch Faserverstärkte Kunststoffe zum Einsatz kommen. Alternativ kann die Aufnahme auch aus Metallen wie zum Beispiel Aluminium oder Magnesium gefertigt sein. Darüber hinaus kann die Aufnahme auch aus Keramik gefertigt sein. Das Kraftübertragungsmittel 50 ist ein Stabelement aus Kunststoff. Alternativ kann das Kraftübertragungsmittel auch faserförmig ausgebildet sein. Des Weiteren kann das Kraftübertragungsmittel auch aus Metall, wie zum Beispiel Aluminium, Magnesium oder Stahl, gefertigt sein.

Der Innenraum der Vorrichtung 20 ist mit einem Füllmedium 30 gefüllt. Bei dem Füllmedium 30 handelt es sich um ein dilatantes Fluid. Alternativ können auch Newtonsche, wie zum Beispiel Silikonöl, als Füllmedium verwendet werden. Darüber hinaus kann auch ein scherverdickender Kunststoff verwendet werden. Dabei liegt der Kunststoff in Pulverform vor. Des Weiteren kann auch Sand als Medium zum Einsatz kommen.

Darüber hinaus ist im Innenraum 24 der Vorrichtung 20 ein erster Körper 40 angeordnet, welcher in der Bewegungsrichtung B relativ zur Aufnahme 20 durch das Füllmedium 30 bewegbar ist. Der erste Körper 40 ist an einem Kraftübertragungsbereich 44 an das Kraftübertragungsmittel 50 gekoppelt, so dass eine von dem Kraftübertragungsmittel 50 ausgehende Kraft auf den ersten Körper 40 übertragen werden kann.

Die Oberfläche des ersten Körpers 40, gegenüber welcher das Füllmedium relativ strömt, wenn der erste Körper 40 in der Bewegungsrichtung B bewegt wird, bildet eine Scherfläche 42. Im Bereich der Scherfläche 42 kommt es zu einer Scherspannungserhöhung durch das Füllmedium, wenn der erste Körper 40 mit einer unphysiologischen Geschwindigkeit durch das Füllmedium bewegt wird.

Ein Spaltmaß S1 stellt den minimalen Abstand zwischen der Scherfläche 42 des ersten Körpers 40 und der Innenfläche 26 der Aufnahme dar. Der erste Körper 40 ist aus Kunststoff gefertigt. Alternativ kann der erste Körper auch aus einem Metall wie zum Beispiel Aluminium gefertigt sein.

Des Weiteren ist im Innenraum 24 der Aufnahme 20 ein zweiter Körper 60 angeordnet, welcher relativ zur Aufnahme 20 in die Bewegungsrichtung B bewegbar ist. Die äußere Umfangsfläche des zweiten Körpers 60 bildet eine Scherfläche 62. Der zweite Körper 60 umfasst Führungsvorsprünge 66, welche den Innenraum der Aufnahme 20 punktförmig kontaktieren können, um den zweiten Körper 60 im Innenraum 24 der Aufnahme 20 beweglich zu führen.

Der kleinste Abstand zwischen der Scherfläche 62 und der Innenfläche 26 der Aufnahme 20 bildet das Spaltmaß S2. Der zweite Körper 60 ist aus Kunststoff gefertigt. Alternativ kann der zweite Körper auch aus einem Metall wie zum Beispiel Aluminium gefertigt sein.

Der erste Körper 40 ist mit dem zweiten Körper 60 über ein elastisches Kopplungselement 70 gekoppelt. Das in den Figuren 1B und 1C gezeigte elastische Kopplungselement 70 ist durch eine Feder gebildet, welche an einem Ende in einem Federsitz 46 des ersten Körpers 40 und am anderen Ende in einem Federsitz 68 des zweiten Körpers 60 gelagert ist. Je nachdem in welche Richtung der Bewegungsrichtung B eine Kraft auf das Kraftübertragungsmittel 50 wirkt, kann der zweite Körper 60 über das Kopplungselement 70 mittels des ersten Körpers 40 gezogen oder geschoben werden. Dabei kann die Feder aus Kunststoff oder aus Metall gefertigt sein. Alternativ kann das elastische Kopplungselement 70 auch in Form eines elastischen Polymers oder Kautschuks ausgebildet sein.

In einer weiteren Alternative ist sind der erste Körper, der zweite Körper und das elastische Kopplungselement einstückig spritzgegossen.

Ferner umfasst der zweite Körper 60 eine Durchlassöffnung 64, durch welche das Füllmedium 30 strömen kann. Wird der zweite Körper 60 entsprechend von einer, von dem Kopplungselement 70 ausgehenden Kraft relativ zur Aufnahme 20 bewegt, kann das scherverdickende Medium 30 sowohl außen im Bereich des Spaltmaßes S2 als auch innen durch die Durchlassöffnung 64 am zweiten Körper entlang strömen.

Die in den Figuren 1A bis 1C gezeigte Vorrichtung 1 ist auf Zugbelastungen ausgelegt. D.h., auch Belastungen, welche aus einem Auseinanderbewegen des Körperbereichs des Anwenders, an dem die Aufnahme 20 befestigt ist und des Körperbereichs des Anwenders, an dem das Kraftübertragungsmittel 50 befestigt ist, resultiert. Wird das Kraftübertragungsmittel 50 aus der Vorrichtung 20 herausgezogen, so zieht es den ersten Körper 40 mit sich, wodurch dieser mittels des Kopplungselements 70 auf den zweiten Körper 60 drückt. Das Kraftübertragungsmittel 50 ist stabförmig ausgeführt und erstreckt sich von dem ersten Körper 40 durch die Durchlassöffnung 64 des zweiten Körpers 60 und schließlich durch die Öffnung 22 der Aufnahme 20. Im Bereich der Öffnung 22 sind in den Figuren 1A und 1B nicht dargestellte Dichtmittel angeordnet, welche den Innenraum 24 der Aufnahme 20 gegenüber der Umgebung abdichten, so dass das Füllmedium 30 im Innenraum 24 der Aufnahme 20 gehalten werden kann.

Nachstehend wird die Funktion der Vorrichtung anhand der Figuren 1B bis 2B beschrieben. Wirkt eine Kraft im Bereich einer physiologischen Geschwindigkeit auf das Kraftübertragungsmittel 50, so dass der erste Körper 40 in Richtung der Öffnung 22 gezogen wird, wird auch der zweite Körper 60 mittels des Kopplungselements 70 in Richtung der Öffnung 22 geschoben. Je nach Größe der Scherfläche 62 und/oder des Spaltmaßes S2 kann ein Schwellwert definiert werden, welcher eine Geschwindigkeit des zweiten Körpers festlegt, bei welcher es aufgrund der Strömung des Füllmedium 30 entlang der Scherfläche 62 zu einer Scherspannungserhöhung kommt, welche keine weitere Bewegung des zweiten Körpers 60 zulässt. Auf diesen Schwellwert kann weiterhin durch die Eigenschaften des elastischen Kopplungselements 70 Einfluss genommen werden. Im Fall des in den Figuren 1A und 1B gezeigten Kopplungselements 70 in Form einer Feder kommt es zu einem Stillstand des zweiten Körpers 60, wenn die, aus der im Bereich der Scherfläche 62 entstehende, resultierende Haltekraft gleich oder größer einer von der Feder ausgehenden Federkraft ist.

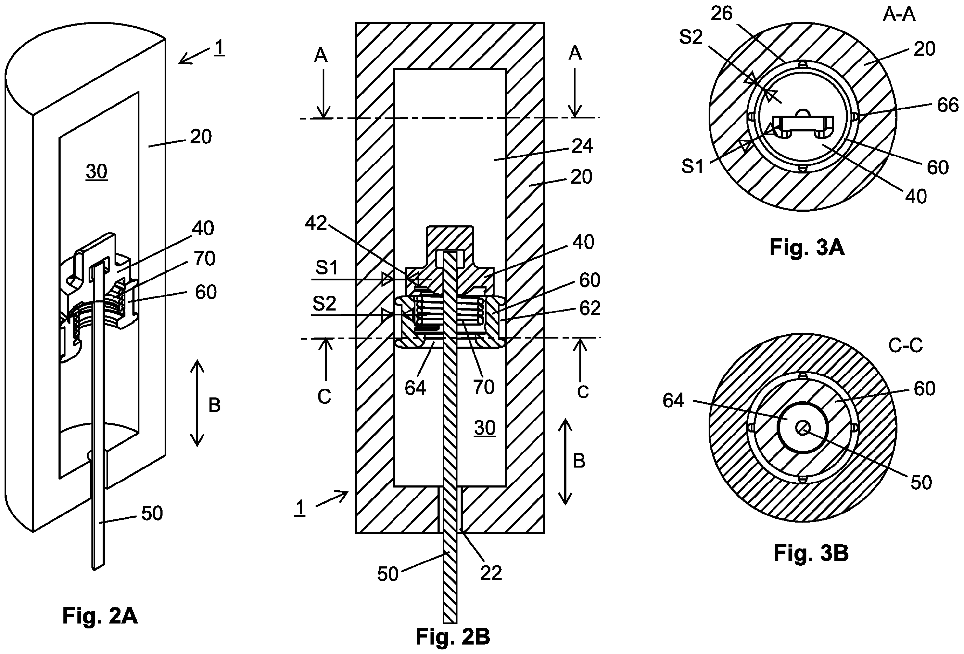

Nachdem der zweite Körper 60 aufgrund der Scherverfestigung im Bereich der Scherfläche 62 blockiert worden ist, bewegt die auf das Kraftübertragungsmittel 50 wirkende Kraft den zweiten Körper 40 weiterhin in Richtung der Öffnung 22. Dabei kann das Füllmedium 30 entlang der Scherfläche 42 des ersten Körpers 40 sowie durch die Durchlassöffnung 64 des zweiten Körpers 60 strömen. Je weiter sich der erste Körper 40 hin zu der Öffnung 22 bewegt, umso kleiner wird der Abstand zwischen dem ersten Körper 40 und dem zweiten Körper 60. Der zweite Körper 40 kann so lange in Richtung der Öffnung 22 bewegt werden, bis der Abstand zwischen dem ersten Körper 40 und dem zweiten Körper 60 geschlossen ist, wie in den Figuren 2A und 2B gezeigt.

In dem in den Figuren 2A und 2B gezeigten Zustand der Vorrichtung 1, kontaktiert der erste Körper 40 den zweiten Körper 60, derart, dass die Durchlassöffnung 64 des zweiten Körpers 60 geschlossen ist. Das sich in dem Innenraum 24 befindliche Füllmedium 30 hat nur noch im Bereich zwischen den Scherflächen 42, 62 des ersten und des zweiten Körpers 40, 60 und der Innenfläche 26 die Möglichkeit, relativ zu dem ersten Körper 40 und dem zweiten Körper 60 zu strömen, sofern es der Grad der Scherverfestigung zulässt.

Um den ersten Körper 40 und den zweiten Körper 60 in Richtung der Öffnung 22 zu bewegen, muss nun eine Haltekraft überwunden werden, welche aus der Interaktion einer Summe der Scherfläche 42 und der Scherfläche 62 mit dem Füllmedium 30 resultiert. Entsprechend ist die Vorrichtung 1 in der Lage, nach dem Schließen der Durchlassöffnung 64 eine signifikant größere Haltekraft bereitzustellen. In der in den Figuren 1A, 1B, 1C, 2A und 2B gezeigten Vorrichtung 1 steigt der geschwindigkeitsabhängige Widerstand der Vorrichtung 1 gegen das Herausziehen des Kraftübertragungsmittels 50 mit dem Schließen der Durchlassöffnung 64 um den Faktor 50. Entsprechend kann die Vorrichtung 1 derart dimensioniert werden, dass bei geöffneter Durchlassöffnung 64 ein physiologischer Kraftaufwand von 20 N nötig ist, um den ersten Körper 40 relativ gegenüber der Aufnahme 20 zu bewegen und bei geschlossener Durchlassöffnung 64 ein Kraftaufwand von 1000 N nötig ist, um den ersten Körper 40 und den zweiten Körper 60 im aneinander liegenden Zustand relativ gegenüber der Aufnahme 20 zu bewegen.

Figur 3A zeigt eine Schnittansicht entlang der Schnittlinie A-A aus Figur 2B. Figur 3A ist zu entnehmen, dass die Führungsvorsprünge 66 den zweiten Körper 60 entlang der Innenfläche 26 der Aufnahme 20 führen. Des Weiteren ist Figur 3A das Spaltmaß S1, welches den kleinsten Abstand zwischen der Scherfläche 42 und der Innenfläche 26 darstellt sowie das Spaltmaß S2, welches den kleinsten Abstand zwischen der Scherfläche 62 und der Innenfläche 26 darstellt, zu entnehmen.

Figur 3B ist eine Schnittansicht entlang der Schnittlinie C-C aus Figur 2B, welcher die Durchlassöffnung 64 in dem zweiten Körper 60 zu entnehmen ist. Ferner zeigt Figur 3B eine konzentrische Anordnung des Kraftübertragungsmittels 50, welches sich durch die Durchlassöffnung 64 hindurch erstreckt, ohne den zweiten Körper 60 zu berühren.

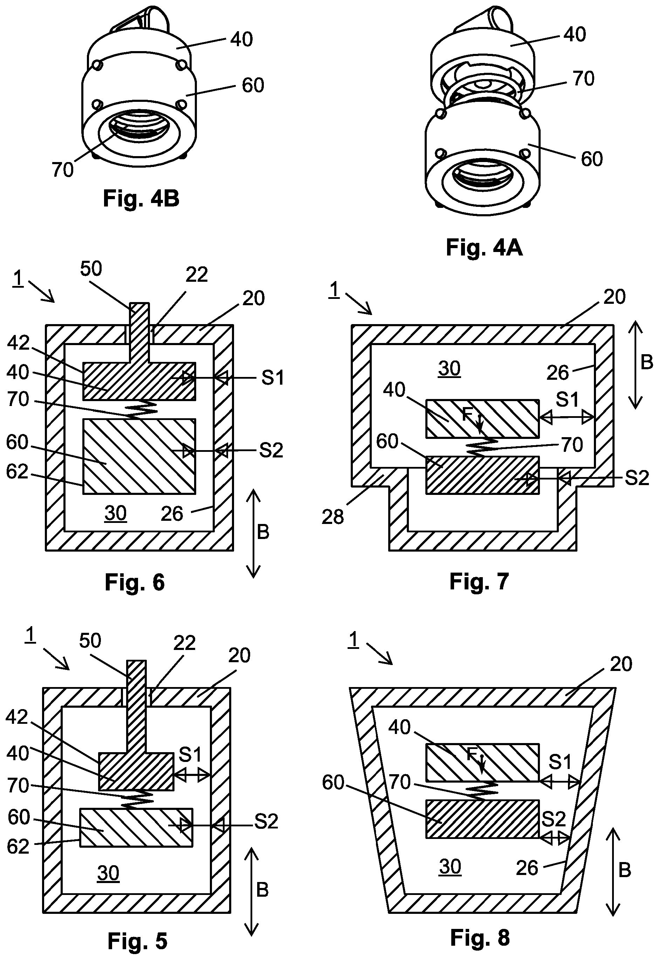

Figur 4A zeigt eine perspektivische Ansicht des ersten Körpers 40, des Kopplungselements 70 und des zweiten Körpers 60 in dem in Figur 1 gezeigten Zustand.

Figur 4B zeigt eine perspektivische Ansicht des ersten Körpers 40, des Kopplungselements 70 und des zweiten Körpers 60 in dem in Figur 2 gezeigten Zustand.

Figur 5 zeigt schematisch eine Vorrichtung 1, welche sich für eine Druckbelastung eignet. Das Kraftübertragungsmittel 50 ist mit dem ersten Körper 40 einstückig verbunden und ragt durch die Öffnung 22 aus der Aufnahme 20 heraus. Wird das Kraftübertragungsmittel 50 in die Aufnahme 20 hineingedrückt, bewegen sich der erste Körper 40 und der zweite Körper 60 von der Öffnung 22 weg. Befindet sich die Geschwindigkeit, mittels welcher sich der erste Körper 40 von der Öffnung 22 wegbewegt, im physiologischen Bereich, wird der zweite Körper 60 über das Kopplungselement 70 von der Öffnung 22 weg geschoben.

Der zweite Körper 60 besitzt ein Spaltmaß S2, welches kleiner ist als das Spaltmaß S1 des ersten Körpers 40. Darüber hinaus ist die Scherfläche 62 des zweiten Körpers 60 größer als die Scherfläche 42 des ersten Körpers 40.

Das Verhältnis der Scherflächen sowie das Verhältnis der Spaltmaße des ersten Körpers 40 und des zweiten Körpers 60 lässt eine Scherspannungserhöhung im Bereich der Scherfläche 62 bei einer Geschwindigkeit in Bewegungsrichtung B auftreten, bei welcher an der Scherfläche 42 noch keine oder eine deutlich geringere Scherspannungserhöhung auftritt. Dadurch ist es möglich, dass der zweite Körper 60 beim Erreichen einer kritischen Geschwindigkeit in Bewegungsrichtung B durch die auf die Scherspannungserhöhung zurückzuführende Haltekraft blockiert wird. In dieser Situation ist die auf den zweiten Körper 60 wirkende Haltekraft gleich oder größer der von dem Kopplungselement 70 ausgehenden, entgegengerichteten elastischen Kraft.

Wird der zweite Körper 60 aufgrund der Scherspannungserhöhung im Bereich der Scherfläche 62 blockiert und wird der erste Körper 40 weiterhin weg von der Öffnung 22 bewegt, so verringert sich der Abstand zwischen dem ersten Körper 40 und dem zweiten Körper 60. In diesem Zustand wird die kritische Geschwindigkeit des ersten Körpers 40 von der Größe der Scherfläche 42 sowie dem Spaltmaß S1 definiert.

Wird durch eine anhaltende, auf das Kraftübertragungsmittel 50 einwirkende Kraft der Abstand zwischen dem ersten Körper 40 und dem zweiten Körper 60 geschlossen, so wird die kritische Geschwindigkeit, bei welcher eine Scherspannungserhöhung eintritt, durch die Summe der Scherflächen 42 und 62 definiert. Der auf das Kraftübertragungsmittel 50 einwirkenden Druckkraft steht nach dem Schließen des Abstands zwischen dem ersten Körper 40 und dem zweiten Körper 60 eine deutlich größere Haltekraft oder Widerstandskraft entgegen.

Die in Figur 6 gezeigte Vorrichtung 1 unterscheidet sich von der in Figur 5 gezeigten Vorrichtung dadurch, dass das Spaltmaß S1 zwischen dem ersten Körper 40 und der Innenfläche 26 dem Spaltmaß S2 zwischen dem zweiten Körper 60 und der Innenfläche 26 gleicht. Darüber hinaus ist die Scherfläche 62 des ersten Körpers 40 deutlich größer als die Schwerfläche 42 des ersten Körpers 40.

Dadurch ist die Geschwindigkeit in Bewegungsrichtung B, bei welcher der zweite Körper 60 aufgrund der Scherspannungserhöhung des Füllmediums 30 im Bereich der Scherfläche 62 blockiert wird, kleiner als die Geschwindigkeit in Bewegungsrichtung B, bei welcher der erste Körper 40 aufgrund der Scherspannungserhöhung des Füllmediums 30 im Bereich der Scherfläche 42 blockiert wird.

Die in Figur 6 gezeigte Vorrichtung 1 verhält sich bei einer Druckbelastung des Kraftübertragungsmittels in das Innere der Aufnahme 20 wie die in Figur 5 gezeigte Vorrichtung.

Figur 7 zeigt eine vereinfachte Darstellung der Aufnahme 20 und des darin angeordneten ersten Körpers 40, welcher über das Kopplungselement 70 mit dem zweiten Körper 60 verbunden ist. Das Profil der Aufnahme 20 weist eine Stufe 28 auf, durch welche die Aufnahme 20 in einen Bereich mit einem großen Durchmesser und einen Bereich mit einem kleinen Durchmesser definiert ist. In Figur 7 ist das Kraftübertragungsmittel der Einfachheit halber nicht dargestellt. Figur 7 zeigt vielmehr einen Pfeil, welcher eine äußere Kraft F präsentiert, welche in Richtung des Bereichs der Aufnahme mit kleinerem Durchmesser zeigt. Der erste Körper 40 und der zweite Körper 60 weisen die gleichen Abmessungen auf. Alternativ können die Abmessungen auch wie in den Figuren 5 und 6 gezeigt, variieren. Der erste Körper 40 und der zweite Körper 60 sind derart angeordnet, dass bei einer Bewegung in Bewegungsrichtung B hin zu dem Bereich der Aufnahme 20 mit dem kleineren Durchmesser der zweite Körper 60 diesen Bereich vor dem ersten Körper 40 erreicht.

Bewegen sich der erste Körper 40 und der zweite Körper 60 durch den Bereich der Aufnahme 20 mit dem größeren Durchmesser, so sind beide Körper durch das Spaltmaß S1 von der Innenfläche 26 der Aufnahme 20 beabstandet. Erreicht der zweite Körper 60 den Bereich der Aufnahme 20 mit kleinerem Durchmesser, so ist der zweite Körper 60 nurmehr durch das Spaltmaß S2 von der Innenfläche 26 der Aufnahme 20 beabstandet. Wie Figur 7 zu entnehmen ist, ist das Spaltmaß S2 kleiner als das Spaltmaß S1. Durch das Erreichen des Bereichs der Aufnahme 20 mit kleinerem Durchmesser, wie in Figur 7 dargestellt, reduziert sich die Geschwindigkeit, bei welcher der zweite Körper 60 aufgrund der Scherverfestigung des Füllmediums 30 blockiert und sich nicht weiter bewegen lässt. Das weitere Verhalten der Vorrichtung entspricht dem der Vorrichtungen aus den Figuren 5 und 6.

Figur 8 zeigt eine vereinfachte Darstellung einer Vorrichtung 1, welche sich von der in Figur 7 gezeigten Vorrichtung dadurch unterscheidet, dass die Fläche der Aufnahme 20 in Bewegungsrichtung B konisch verläuft. Aufgrund der konischen Ausgestaltung der Aufnahme 20 ist bei gleicher Abmessung des ersten Körpers 40 und des zweiten Körpers 60 das Spaltmaß S1 des ersten Körpers 40 stets größer als das Spaltmaß S2 des zweiten Körpers 60. Das Verhalten der in Figur 8 gezeigten Vorrichtung 1 durch Einleiten einer Kraft F auf den ersten Körper entspricht dem Verhalten der Vorrichtungen aus den Figuren 5, 6 und 7.

Um die in den vorstehenden Figuren dargestellten Vorrichtungen wieder in eine Ausgangsposition zu bringen, können Rückstellmittel vorgesehen sein. Diese Rückstellmittel können beispielsweise elastisch ausgeführt sein und den ersten Körper mit der in Bewegungsrichtung gegenüberliegenden Seite der Aufnahme verbinden. Wird der erste Körper durch eine einwirkende Kraft aus der Ausgangsposition ausgelenkt, wird das elastische Rückstellmittel gedehnt. Lässt die äußere Kraft sowie die Haltekraft der Scherverfestigung nach, kann das elastische Rückstellmittel aufgrund der zuvor erfahrenen Dehnung den ersten Körper, das Kopplungselement und den zweiten Körper wieder in die Ausgangsposition befördern.

Die Vorrichtung kann beispielsweise in folgenden Produkten Anwendung finden: Schuhe, Hosen, Jacken, Oberhemden, Strümpfe, Handschuhe, Protektoren, Schutzkleidung, Prothesen, Bandagen, Orthesen, Tapes, Helme, Schienbeinschoner, Stiefel, Verbände, etc.

Soweit anwendbar, können alle einzelnen Merkmale, die in den einzelnen Ausführungsbeispielen dargestellt sind, miteinander kombiniert und/oder ausgetauscht werden, ohne den Bereich der Erfindung zu verlassen.

### Bezugszeichenliste

- 1: Vorrichtung

- 20: Aufnahme
- 22: Öffnung
- 24: Innenraum
- 26: Innenfläche
- 28: Stufe

- 30: Füllmedium

- 40: Erster Körper
- 42: Scherfläche
- 44: Kraftübertragungsbereich
- 46: Federsitz

- 50: Kraftübertragungsmittel

- 60: Zweiter Körper
- 62: Scherfläche
- 64: Durchlassöffnung
- 66: Führungsvorsprung
- 68: Federsitz

- 70: Kopplungselement

- S1: Spaltmaß
- S2: Spaltmaß
- B: Bewegungsrichtung
- F: Kraft

## Patentansprüche

1. Vorrichtung (1) zur Stabilisierung von Körpergelenken, umfassend:
eine Aufnahme (20), wobei die Aufnahme (20) mit einem Füllmedium (30) gefüllt ist, wobei das Füllmedium ein Fluid ist,
einen ersten Körper (40) zum Interagieren mit dem Füllmedium (30), wobei der erste Körper in der Aufnahme (20) verschiebbar angeordnet ist,
ein Kraftübertragungsmittel (50) zum Übertragen einer äußeren Kraft auf den ersten Körper (40),
einen zweiten Körper (60) zum Interagieren mit dem Füllmedium (30), welcher in der Aufnahme (20) verschiebbar angeordnet ist,
wobei eine Oberfläche des ersten Körpers (40), gegenüber der das Füllmedium (30) eine relative Strömungsbewegung ausführt, wenn der erste Körper (40) aufgrund einer äußeren Kraft bewegt wird, eine erste Scherfläche (42) bildet und eine Oberfläche des zweiten Körpers (62), gegenüber der das Füllmedium (30) eine relative Strömungsbewegung ausführt, wenn der zweite Körper (60) aufgrund einer äußeren Kraft bewegt wird, eine zweite Scherfläche (62) bildet,
wobei der zweite Körper über ein Kopplungselement (70) elastisch mit dem ersten Körper (40) gekoppelt ist, wobei das Kopplungselement (70) ein Federelement umfasst,
wobei der zweite Körper (60) und/oder der erste Körper (40) mindestens eine Durchlassöffnung (64) aufweisen, durch welche das Füllmedium (30) strömen kann, und
wobei der erste Körper (40) einen Ventilkörper bildet und der zweite Körper (60) einen Ventilsitz bildet, so dass ein Fluss des Füllmediums (30) durch die Durchlassöffnung (64) in Abhängigkeit von der Ventilstellung zugelassen oder unterbunden werden kann,
**dadurch gekennzeichnet, dass**
je nach Größe eines Spaltmaßes (S2) zwischen dem zweiten Körper (60) und der Aufnahme (20) und/oder einer Federkraft des Kopplungselements (70) und/oder der zweiten Scherfläche (62) ein Schwellwert definiert wird, welcher eine Geschwindigkeit des zweiten Körpers festlegt, bei welcher es aufgrund der Strömung des Füllmediums (30) entlang der zweiten Scherfläche (62) zu einer Scherspannungserhöhung kommt, welche keine weitere Bewegung des zweiten Körpers (60) zulässt.

2. Vorrichtung (1) gemäß Anspruch 1, **dadurch gekennzeichnet, dass** die erste Scherfläche (42) und die zweite Scherfläche (62) eine unterschiedliche Größe aufweisen.

3. Vorrichtung (1) gemäß Anspruch 1 oder 2, **dadurch gekennzeichnet, dass** die erste Scherfläche (42) kleiner ist als die zweite Scherfläche (62).

4. Vorrichtung (1) gemäß einem der vorhergehenden Ansprüche, **dadurch gekennzeichnet, dass** ein Spaltmaß (S1) zwischen dem ersten Körper (40) und der Aufnahme (20) unterschiedlich zu dem Spaltmaß (S2) zwischen dem zweiten Körper (60) und der Aufnahme (20) ist.

5. Vorrichtung (1) gemäß Anspruch 4, **dadurch gekennzeichnet, dass** das Spaltmaß (S1) zwischen dem ersten Körper (40) und der Aufnahme (20) größer als das Spaltmaß (S2) zwischen dem zweiten Körper (60) und der Aufnahme (20) ist.

6. Vorrichtung (1) gemäß einem der vorhergehenden Ansprüche, **dadurch gekennzeichnet, dass** das Kopplungselement (70) aus einem Material mit einem temperaturabhängigen Elastizitätsmodul gefertigt ist.

7. Vorrichtung (1) gemäß einem der vorhergehenden Ansprüche, **dadurch gekennzeichnet, dass** die Durchlassöffnung (64) mittels des ersten Körpers (40) und/oder des zweiten Körpers (60) verschließbar ist, so dass ein Fluss des Füllmediums (30) durch die Durchlassöffnung (64) unterbunden werden kann.

8. Vorrichtung (1) gemäß einem der vorhergehenden Ansprüche, **dadurch gekennzeichnet, dass** das Kraftübertragungsmittel (50) zum Übertragen einer äußeren Kraft einstückig mit dem ersten Körper (40) ausgebildet ist.

9. Vorrichtung (1) gemäß einem der vorhergehenden Ansprüche, **dadurch gekennzeichnet, dass** der erste Körper (40) mittels des Kopplungselements (70) eine Druckkraft und/oder eine Zugkraft auf den zweiten Körper (60) ausüben kann.

10. Vorrichtung (1) gemäß einem der vorhergehenden Ansprüche, **dadurch gekennzeichnet, dass** die Größe der Scherfläche (42) des ersten Körpers (40), und die Größe der Scherfläche (62) des zweiten Körpers (60) derart konfiguriert sind, dass wenn eine äußere Kraft mit einer Geschwindigkeit unterhalb des Schwellwerts auf den ersten Körper (40) wirkt, der erste Körper (40) und der zweite Körper (60) nahezu geleichmäßig durch das Füllmedium (30) bewegbar sind, und
dass, wenn eine äußere Kraft mit einer Geschwindigkeit größer gleich des Schwellwerts auf den ersten Körper (40) wirkt, der erste Körper (40) und der zweite Körper (60) relativ zueinander bewegbar sind.

11. Vorrichtung (1) gemäß einem der vorhergehenden Ansprüche, **dadurch gekennzeichnet, dass** das Füllmedium (30) eine newtonsche Flüssigkeit ist.

12. Vorrichtung (1) gemäß Anspruch 11, **dadurch gekennzeichnet, dass** das Füllmedium Silikonöl ist.

13. Vorrichtung (1) gemäß einem der Ansprüche 1 bis 10, **dadurch gekennzeichnet, dass** das Füllmedium (30) scherverdickend ist.

## Claims

1. A device (1) for stabilizing body joints, comprising:
a receptacle (20), wherein the receptacle (20) is filled with a filling medium (30), wherein the filling medium is a fluid,
a first body (40) for interacting with the filling medium (30), wherein the first body is slidably arranged in the receptacle (20),
a force transmission means (50) for transmitting an external force to the first body (40),
a second body (60) for interacting with the filling medium (30), which is slidably arranged in the receptacle (20),
wherein a surface of the first body (40), relative to which the filling medium (30) performs a relative flow movement when the first body (40) is moved due to an external force, forms a first shear surface (42) and a surface of the second body (62), relative to which the filling medium (30) performs a relative flow movement when the first body (40) is moved due to an external force, forms a second shear surface (62),
wherein the second body is elastically coupled to the first body (40) via a coupling element (70), the coupling element (70) comprising a spring element,
wherein the second body (60) and/or the first body (40) having at least one passage opening (64), through which the filling medium (30) can flow, and
wherein the first body (40) forms a valve body and the second body (60) forms a valve seat, so that a flow of the filling medium (30) through the passage opening (64) can be permitted or prevented depending on the valve position,
**characterized in in that**,
depending on the size of a gap size (S2) between the second body (60) and the receptacle (20) and/or a spring force of the coupling element (70) and/or the second shear surface (62), a threshold value is defined which determines a speed of the second body at which, due to the flow of the filling medium (30) along the second shear surface (62), leads to an increase in shear stress which does not permit any further movement of the second body (60).

2. Device (1) according to claim 1, **characterized in that** the first shear surface (42) and the second shear surface (62) have a different size.

3. Device (1) according to claim 1 or 2, **characterized in that** the first shear surface (42) is smaller than the second shear surface (62).

4. Device (1) according to any of the preceding claims, **characterized in that** a gap size (S1) between the first body (40) and the receptacle (20) is different from the gap size (S2) between the second body (60) and the receptacle (20).

5. Device (1) according to claim 4, **characterized in that** the gap size (S1) between the first body (40) and the receptacle (20) is larger than the gap size (S2) between the second body (60) and the receptacle (20).

6. Device (1) according to any of the preceding claims, **characterized in that** the coupling element (70) is made of a material with a temperature-dependent modulus of elasticity.

7. Device (1) according to any of the preceding claims, **characterized in that** the passage opening (64) is closable by means of the first body (40) and/or the second body (60), so that a flow of the filling medium (30) through the passage opening (64) may be prevented.

8. Device (1) according to any of the preceding claims, **characterized in that** the force transmission means (50) for transmitting an external force is formed integrally with the first body (40).

9. Device (1) according to any of the preceding claims, **characterized in that** the first body (40) can exert a compressive force and/or a tensile force on the second body (60) by means of the coupling element (70).

10. Device (1) according to any of the preceding claims, **characterized in that** the size of the shear surface (42) of the first body (40) and the size of the shear surface (62) of the second body (60) are configured such that, when an external force acts on the first body (40) at a speed below the threshold value, the first body (40) and the second body (60) are movable almost uniformly through the filling medium (30), and
that when an external force acts on the first body (40) at a speed larger than or equal to the threshold value, the first body (40) and the second body (60) are movable relative to one another.

11. Device (1) according to any of the preceding claims, **characterized in that** the filling medium (30) is a Newtonian fluid.

12. Device (1) according to claim 11, **characterized in that** the filling medium is silicone oil.

13. Device (1) according to any of claims 1 to 10, **characterized in that** the filling medium (30) is shear-thickening.

## Revendications

1. Dispositif (1) pour la stabilisation des articulations du corps, comprenant:
un réceptacle (20), le réceptacle (20) étant rempli d'un milieu de remplissage (30), le milieu de remplissage étant un fluide,
un premier corps (40) destiné à interagir avec le milieu de remplissage (30), le premier corps étant disposé de manière déplaçable dans le réceptacle (20), un moyen de transmission de force (50) destiné à transmettre une force extérieure au premier corps (40),
un deuxième corps (60) destiné à interagir avec le milieu de remplissage (30), qui est disposé de manière déplaçable dans le réceptacle (20),
une surface du premier corps (40), par rapport à laquelle le milieu de remplissage (30) effectue un mouvement d'écoulement relatif lorsque le premier corps (40) est déplacé en raison d'une force extérieure, forme une première surface de cisaillement (42) et une surface du deuxième corps (62), par rapport à laquelle le milieu de remplissage (30) effectue un mouvement d'écoulement relatif lorsque le deuxième corps (60) est déplacé en raison d'une force extérieure, forme une deuxième surface de cisaillement (62),
le deuxième corps étant couplé élastiquement au premier corps (40) par l'intermédiaire d'un élément de couplage (70), l'élément de couplage (70) comprenant un élément de ressort,
le deuxième corps (60) et/ou le premier corps (40) présentant au moins une ouverture de passage (64), à travers laquelle le milieu de remplissage (30) peut s'écouler, et
dans lequel le premier corps (40) forme un corps de vanne et le deuxième corps (60) forme un siège de vanne, de sorte qu'un écoulement du milieu de remplissage (30) à travers l'ouverture de passage (64) peut être autorisé ou empêché en fonction de la position de la vanne, **caractérisé en ce que**,
en fonction de la taille d'une mesure de fente (S2) entre le deuxième corps (60) et le réceptacle (20) et/ou d'une force de ressort de l'élément de couplage (70) et/ou de la deuxième surface de cisaillement (62), une valeur seuil est définie, qui elle définie une vitesse du deuxième corps à laquelle, en raison de l'écoulement du milieu de remplissage (30) le long de la deuxième surface de cisaillement (62), il se produit une augmentation de la tension de cisaillement qui n'autorise plus aucun autre mouvement du deuxième corps (60).

2. Dispositif (1) selon la revendication 1, **caractérisé en ce que** la première surface de cisaillement (42) et la deuxième surface de cisaillement (62) présentent une taille différente.

3. Dispositif (1) selon la revendication 1 ou 2, **caractérisé en ce que** la première surface de cisaillement (42) est plus petite que la deuxième surface de cisaillement (62).

4. Dispositif (1) selon l'une des revendications précédentes, **caractérisé en ce qu'**une mesure de fente (S1) entre le premier corps (40) et le réceptacle (20) est différente par rapport à la mesure de fente (S2) entre le deuxième corps (60) et le réceptacle (20).

5. Dispositif (1) selon la revendication 4, **caractérisé en ce que** la mesure de fente (S1) entre le premier corps (40) et le réceptacle (20) est supérieure à la mesure de fente (S2) entre le deuxième corps (60) et le réceptacle (20).

6. Dispositif (1) selon l'une des revendications précédentes, **caractérisé en ce que** l'élément de couplage (70) est réalisé dans un matériau présentant un module d'élasticité dépendant de la température.

7. Dispositif (1) selon l'une des revendications précédentes, **caractérisé en ce que** l'ouverture de passage (64) peut être verrouillée au moyen du premier corps (40) et/ou du deuxième corps (60), de sorte qu'un écoulement du milieu de remplissage (30) à travers l'ouverture de passage (64) peut être empêché.

8. Dispositif (1) selon l'une des revendications précédentes, **caractérisé en ce que** le moyen de transmission de force (50) destiné à transmettre une force extérieure est formé d'une seule pièce avec le premier corps (40).

9. Dispositif (1) selon l'une des revendications précédentes, **caractérisé en ce que** le premier corps (40) est apte à exercer une force de compression et/ou une force de traction sur le deuxième corps (60) au moyen de l'élément de couplage (70).

10. Dispositif (1) selon l'une des revendications précédentes, **caractérisé en ce que** la taille de la surface de cisaillement (42) du premier corps (40), et la taille de la surface de cisaillement (62) du deuxième corps (60) sont configurées de telle sorte que lorsqu'une force extérieure agit sur le premier corps (40) à une vitesse inférieure à la valeur de seuil, le premier corps (40) et le deuxième corps (60) sont déplaçables presque uniformément à travers le milieu de remplissage (30), et
que lorsqu'une force extérieure agit sur le premier corps (40) à une vitesse supérieure ou égale à la valeur de seuil, le premier corps (40) et le deuxième corps (60) sont déplaçables l'un par rapport à l'autre.

11. Dispositif (1) selon l'une des revendications précédentes, **caractérisé en ce que** le milieu de remplissage (30) est un liquide newtonien.

12. Dispositif (1) selon la revendication 11, **caractérisé en ce que** le milieu de remplissage est une huile de silicone.

13. Dispositif (1) selon l'une des revendications 1 à 10, **caractérisé en ce que** le milieu de remplissage (30) est épaississant par cisaillement.
